## Europäisches Patentamt
## European Patent Office
## Office européen des brevets

(19)

(11) Numéro de publication: **0 041 052 B1**

(12) **FASCICULE DE BREVET EUROPEEN**

(45) Date de publication du fascicule du brevet:
14.11.84

(51) Int. Cl.³: **A 61 F 1/04**, A 61 F 1/08

(21) Numéro de dépôt: **81830071.7**

(22) Date de dépôt: **12.05.81**

(54) **Prothèse articulée pour membre inférieur.**

(30) Priorité: **28.05.80 IT 945080**
**27.02.81 IT 935081**

(43) Date de publication de la demande:
**02.12.81 Bulletin 81/48**

(45) Mention de la délivrance du brevet:
**14.11.84 Bulletin 84/46**

(84) Etats contractants désignés:
**AT BE CH DE FR GB LI LU NL SE**

(56) Documents cités:
**DE - C - 622 314**
**DE - C - 742 947**
**GB - A - 414 317**

(73) Titulaire: **Serri, Roberto, Via Signorini 21,**
**I-50018 Scandicci (FI) (IT)**

(72) Inventeur: **Serri, Roberto, Via Signorini 21,**
**I-50018 Scandicci (FI) (IT)**

(74) Mandataire: **Martini, Lazzaro, Ufficio Brevetti Ing.**
**Lazzaro Martini Via Brunelleschi, 1, I-50123 Firenze (IT)**

## Description

L'invention concerne une prothèse articulée pour membre inférieur comprenant des articulations au niveau du genou et du pied qui sont synchronisées, l'articulation du genou ayant des axes de rotation qui sont décalés en arrière de l'axe longitudinal de la prothèse, des moyens qui provoquent le redressement du genou pour l'avancement spontané de la jambe lorsque le pied est soulevé du sol et des moyens supplémentaires pour la rotation contrôlée du pied lorsque le pied est appuyé à terre et le poids du corps porte sur celui-ci pour permettre d'avancer l'autre jambe.

Comme nous le savons, dans les prothèses traditionnelles pour membre inférieur, un cuissard est relié pivotant verticalement par rapport à la partie inférieure de la prothèse par l'intermédiaire d'un axe horizontal coupant l'axe longitudinal vertical de la prothèse et l'extrémité inférieure du cuissard est en forme de genou et constitue un élément de butée pour la partie inférieure du membre apte à empêcher une rotation non naturelle.

En particulier on connait par les brevets DE-A-742 947 et GB-A-414 317 une articulation du genou comprenant des éléments à dentures engrenant ensemble.

Une telle structure présente une relative instabilité lorsqu'elle doit supporter tout le poids du corps par le fait que l'axe de l'articulation du genou coupant l'axe longitudinal de la prothèse est responsable d'une rotation inattendue et incontrôlée de l'articulation. En outre, dans lesdites prothèses, le pied est relié pivotant verticalement par rapport à la partie supérieure de la prothèse par l'intermédiaire d'un axe horizontal, interposant en outre, au niveau du talon, des dispositifs élastiques qui agissent en opposition en un point d'arrêt prévu dans la partie antérieure du pied. Le dit point d'arrêt antérieur fixe empêche toute rotation vers le haut de la pointe du pied lorsque le pied est appuyé sur le sol pendant qu'on fait avancer l'autre jambe. En outre, étant donné que l'articulation du pied et celle du genou sont indépendantes entre elles, pendant le mouvement de retour en avant de la prothèse, la pointe du pied est dirigée vers le sol, de sorte qu'il est nécessaire pour avancer, de se soulever sur la pointe de l'autre pied.

En particulier on connait par le brevet DE-A-622 314 une prothèse articulée pour membre inférieur dont l'articulation du genou comprend deux éléments pivotants autour d'un axe horizontal coupant l'axe longitudinal de la prothèse et une bielle dont les extrémités sont articulées autour d'axes parallèles décalés en arrière de l'axe d'articulation des dits éléments, avec pour fonction d'empêcher toute rotation en avant de la prothèse au niveau du genou; mais une telle structure est assez complexe, lourde et bruyante. En outre, l'articulation du pied de la prothèse décrite dans le dit brevet est asservie à l'articulation du genou de telle façon qu'elle empêche toute rotation vers le haut de la pointe du pied lorsqu'il est soulevé du sol.

La présente invention a pour but de remédier à ces inconvénients et, en outre, a pour but d'obtenir, pendant la déambulation, le redressement du genou de la prothèse pour l'avancement spontané de la jambe.

L'invention concerne une prothèse articulée pour membre inférieur pourvue d'articulations au niveau du genou et du pied respectivement situées aux extrémités supérieure et inférieure d'un montant vertical tubulaire surmonté d'un dispositif de fixation à la cuisse, prothèse dont l'articulation du genou comprend deux éléments coopérants entre eux par l'intermédiaires de parties respectives pivotantes autour d'axes horizontaux, parallèles entre eux et espacés et dont l'articulation du pied comprend deux éléments reliés par l'intermédiaire d'une charnière horizontale et deux ressorts de compression parallèles à l'axe du montant et exerçant des efforts antagonistes par rapport à la charnière les articulations du genou et du pied étant assemblées entre elles par l'intermédiaire de dispositifs qui comprennent le montant tubulaire, un tube logé dans le montant, une biellette reliée à un support, un tirant logé dans le montant tubulaire et un piston reliée au tirant et coulissant à l'intérieur du tube, prothèse caractérisée

– en ce que les éléments de l'articulation du genou ont la forme de leviers superposés, articulés en arrière de l'axe YY de la prothèse sur le support qui est tubulaire et commun aux deux éléments, les bras des leviers étant reliés au sommet du montant et à la base du dispositif de fixation à la cuisse, et les extrémités de ces leviers qui entourent les axes d'articulation étant pourvues de dentures engrénant ensemble;

– en ce que les deux ressorts sont disposés l'un devant et l'autre derrière la charnière, le ressort arrière étant plus faible que le ressort avant et intercalé entre les deux éléments de l'articulation de manière à les écarter,

– en ce que le piston des dispositifs d'assemblage des articulations du genou et du pied comporte un appendice faisant saillie vers l'avant du montant auquel est reliée une tige qui est parallèle au tirant et porte le ressort qui est situé en avant de la charnière de l'articulation du pied et qui est logé dans une cavité traversant l'élément supérieur de l'articulation du pied et dont la base s'appuie sur l'élément inférieur de elle-ci,

– en ce que l'extrémité supérieure du tirant est reliée au support tubulaire par la biellette et,

– en ce qu'un ressort de compression logé dans le tube sollicite le piston vers l'articulation du pied.

L'invention résoud le problème consistant à réaliser un équilibre stable de la prothèse lorsqu'elle supporte le poids du corps et en combinaison, à réaliser, pendant la déambulation, une synchronisation précise, complète et silencieuse entre la flexion du genou et le soulèvement de la pointe du pied et en outre à réaliser le redressement successif du genou pour l'avancement spontané de la jambe lorsque le pied est soulevé du sol et enfin à réaliser la rotation contrôlée du pied quand il est appuyé à terre et supporte le poids du corps pour permettre d'avancer l'autre jambe. Ce résultat a été obtenu en réalisant l'articulation du genou avec deux leviers pivotant verticalement autour de deux axes horizontaux parallèles situés en arrière de l'axe longitudinal de la prothèse et en outre qui coopèrent entre eux le long de parties de surfaces dentées; en rendant rigide la jonction entre les articulations du genou et du pied en associant les éléments pour cette jonction rigide avec un premier ressort antagoniste qui se contracte lors de la flexion du genou et se détend ensuite en provoquant l'avancement spontané de la jambe, avec un second ressort qui se détend lors de la flexion du genou en provoquant le soulèvement de la pointe du pied et avec un troisième ressort qui se contracte lors de la compression du pied et se détend ensuite lors du redressement du genou.

Les avantages obtenus grâce à l'invention consistent essentiellement en ce que la prothèse, dans la position verticale, assure le maximum de stabilité et de sécurité; le soulèvement correct de la pointe du pied lors de la flexion de l'articulation du genou élimine tout choc contre le sol et donc toute résistance à l'avancement de la jambe même sur un terrain en montée en évitant en même temps de devoir se soulever sur l'autre pied; l'avancement spontané de la jambe après la flexion du genou réduit fortement l'effort de déambulation; la possibilité de faire tourner le pied qui est appuyé à terre avec la compression de la pointe lorsque la prothèse supporte le poids du corps et qu'on fait avancer l'autre jambe; une déambulation silencieuse, fluide et harmonieuse.

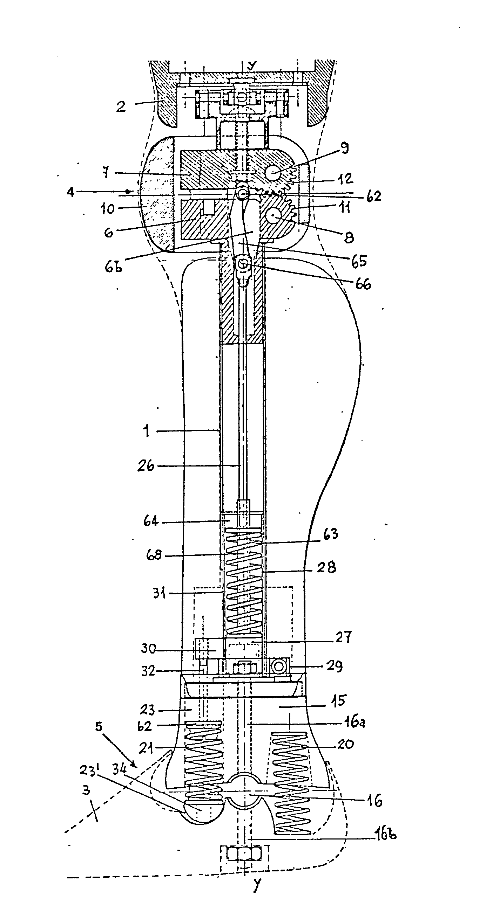

L'invention sera exposée ci-après plus en détail à l'aide du dessin annexé.

La figure unique représente une vue de côté en coupe d'une prothèse articulée conformément à la présente invention.

Réduite à sa structure essentielle et se référant au dessin annexé, une prothèse articulée pour membre inférieur conforme à l'invention, comprend:

– Un montant tubulaire (1), aux extrémités supérieures et inférieures duquel se situent les dispositifs d'articulation (4) et (5) du genou et du pied, respectivement.

– Un dispositif supérieur (2) de fixation de la prothèse à la cuisse, relié à l'articulation (4) du genou.

– Un élément inférieur (3) qui forme le pied, relié à l'articulation (5) du pied.

– Un dispositif d'articulation (4) du genou, constitués des éléments (6) et (7) reliés au montant (1) et au dispositif d'appui (2) et pivotant autour d'axes (8, 9) horizontaux parallèles entre eux et qui sont soutenus par un support (10): les dits axes (8–9) sont décalés en arrière de l'axe (yy) du montant (1); les extrémités des éléments (6–7) entourant les axes (8–9) ont une forme de cylindres dentés avec les dentures (11–12) qui engrènent entre elles de façon à ce que les éléments (6–7) coopèrent le long de ces parties de surfaces dentées cylindriques; le support (10) consiste en un corps tubulaire servant de logement aux éléments (6) et (7) et de support des axes (8) et (9) par l'intermédiaire desquels la prothèse supporte le poids du corps.

– Un dispositif d'articulation (5) du pied constitué des éléments (3) et (15), ce dernier étant fixé au montant, les dits éléments étant reliés entre eux par l'intermédiaire d'une charnière cylindrique (16) horizontale coupant l'axe (yy) du montant (1) et pourvue de deux tiges filetées (16a) et (16b) coaxiales à l'axe (yy) pour son assemblage au montant (1) et au pied (3) respectivement.

– Une biellette (65) coudée, fixée à sa partie supérieure tournée vers la partie postérieure de la jambe par un axe (62) au support (10) de l'articulation du genou, et s'étendant à travers une ouverture (6b) au-delà de l'élément mobile (6) de l'articulation du genou et jusqu'à l'intérieur de ce montant (1).

– Un tirant (26) rigide, interne au montant (1) et aligné avec la biellette (65) avec son extrémité supérieure fixée par un axe (66) à l'extrémité inférieure de la biellette (65) et avec son extrémité inférieure fixée, par filetage, en tête d'un tube (68) coaxial au montant (1).

– Ce tube (68) dont la partie supérieure passe dans un trou axial d'un bloc obturateur (64) fixé en tête d'un tube (28) et dont la partie inférieure est solidaire d'un piston (27) coulissant dans le tube (28), un appendice (30) de ce piston faisant transversalement saillie hors du montant (1) à travers une ouverture (31) longitudinale ménagée dans le montant (1) et dans le tube (28).

– Un ressort antagoniste hélicoïdal cylindrique de compression (63) placé sur le tube (68) et se trouvant entre le bloc obturateur (64) et le piston (27).

– Une tige (32) parallèle au tirant (26) fixée à son extrémité supérieure par un filetage à l'extrémité de l'appendice (30) et, à son extrémité inférieure à un petit plateau (62) placé librement à l'intérieur d'une cavité (23) cylindrique, traversant verticalement l'élément fixe (15) de l'articulation du pied.

– Un ressort hélicoïdal cylindrique de compression (21) inséré mobile dans cette cavité (23) et accroché à sa partie supérieure au plateau (62) et à sa partie inférieure à une tête (34) placée dans une encoche (23') de l'élément mobile (3) de l'articulation du pied.

– Un ressort hélicoïdal cylindrique de compression (20) inséré entre les deux éléments (3–15) de l'articulation du pied, arrière de la charnière (16) et d'une force plus faible que celle du ressort (21).

Le fonctionnement est le suivant:

En commençant à porter en avant, dans le sens de la marche, la cuisse et avec elle l'élément (2) de fixation de la prothèse, l'élément mobile (6) de l'articulation du genou s'écarte vers le bas de l'élément fixe (7) du fait de la rotation de la partie dentée (11) sur la partie dentée fixe (12); en même temps, la biellette (65) et le tirant (26) soulèvent le piston (27) avec l'appendice (30) et la tige (32) avec le ressort (21) en provoquant la compression du ressort antagoniste (63) et le soulèvement de la pointe de l'élément mobile (3) du pied, du fait de la poussée produite par le ressort (20); en continuant le soulèvement de la cuisse et une fois soulevé le pied du sol, le ressort antagoniste (63) se détend en faisant avancer le montant (1) jusqu'à l'arrêt complet de l'articulation du genou; après quoi, une fois appuyé le pied à terre et le poids du corps agissant sur la partie antérieure de celui-ci sans pour autant plier l'articulation du genou, le ressort (21) se contracte en contrôlant la rotation de l'élément mobile (3) de l'articulation du pied et en permettant de faire avancer l'autre jambe.

**Revendications**

1. Prothèse articulée pour membre inférieur pourvue d'articulations (4–5) au niveau du genou et du pied, respectivement situées aux extrémités supérieure et inférieure d'un montant (1) tubulaire vertical, surmonté d'un dispositif (2) de fixation à la cuisse, prothèse dont l'articulation (4) du genou comprend deux éléments (6–7) coopérants entre eux par l'intermédiaires de parties respectives pivotantes autour d'axes (8–9) horizontaux, parallèles entre eux et espacés et dont l'articulation (5) du pied comprend deux éléments superposés (3–15) reliés par une charnière (16) horizontale et deux ressorts de compression (20–21) parallèles à l'axe du montant (1) mais exerçant des efforts antagonistes par rapport à la charnière (16), les articulations (4–5) du genou et du pied étant assemblées entre elles par l'intermédiaire de dispositifs qui comprennent le montant (1), un tube (28), logé dans le montant (1) une biellette (65) reliée à un support (10), un tirant 26 inséré dans le montant tubulaire 1, un piston 27 relié au tirant (26) et coulissant dans le tube (28), caractérisée en ce que:
   – les éléments (6–7) de l'articulation 4 du genou ont la forme de leviers superposés, articulés autour d'axes (8, 9) en arrière de l'axe YY de la prothèse sur le support (10) qui est tubulaire et commun aux deux éléments, les bras des dits leviers étant respectivement reliés au sommet du montant (1) et à la base du dispositif (2) de fixation à la cuisse et les extrémités des dits leviers qui entourent les axes d'articulation (8–9) étant pourvues de dentures (11–12) engrenant ensemble;
   – les deux ressorts (20–21) de l'articulation (5) du pied sont placés l'un (21) devant et l'autre (20) derrière la charnière (16), le ressort arrière (20) étant plus faible que le ressort avant (21) et intercalé entre les deux éléments (3, 15) de manière à les écarter,
   – le piston (27) des dispositifs d'assemblage des articulations (4–5) du genou et du pied entre elles comporte un appendice (30) faisant saillie vers l'avant du montant (1) et auquel est reliée une tige (32) qui est parallèle au tirant (26) et porte le ressort (21) qui est situé en avant de la charnière (16) de l'articulation (5) du pied, qui est logé dans une cavité (23) traversant l'élément supérieur (15) de l'articulation (5) du pied et dont la base s'appuie sur l'élément inférieur (3) de celle-ci,
   – l'extrémité supérieure du tirant (26) est reliée au support tubulaire (10) par la biellette (65), et
   – un ressort de compression (63) logé dans le tube (28) sollicite le piston (27) vers l'articulation (5) du pied.

2. Prothèse selon la revendication 1, caractérisée en ce que ladite biellette (65) est coudée avec son extrêmité supérieure tournée vers la partie postérieure de la jambe, en ce qu'elle passe à travers une ouverture (6b) de l'élément inférieur (6) de l'articulation du genou, en ce que la tête de la biellette (65) est articulée au support (10) autour d'un axe (62) parallèle à l'axe (8) du levier inférieur (6) et est située dans le plan de l'axe longitudinal (yy) de la jambe et en ce que le pied de la biellette est logé à l'intérieur du montant (1) de sorte que la biellette (65) soit alignée avec le tirant (26).

3. Prothèse selon la revendication 1, caractérisée par le fait que l'extrémité supérieure du tirant (26) est articulée à la biellette (65) autour d'un axe (66) parallèle à l'axe (8) et que son extrémité inférieure est vissée à la partie supérieure d'un tube (68) dont la base est fixée au piston (27).

4. Prothèse selon la revendication 1, caractérisée par le fait que ladite tige (32) est vissée dans l'appendice (30).

5. Prothèse selon la revendication 1, caractérisée par le fait que le dit ressort (21) est fixé à sa partie supérieure à un plateau (62) de la tige (32) et, à sa partie inférieure, à une tête (34) placée libre dans une encoche (23') de l'élément (3) de l'articulation du pied.

**Patentansprüche**

1. Gegliederte Prothese für die untersten Glieder, welche mit in Knie- und Fusshöhe befindlichen Artikulationen (4–5) versehen ist, die an den oberen bzw. unteren Enden eines senkrechten röhrenförmigen Pfostens (1) gelegen sind, auf welchem eine mit dem Schenkel in Verbindung stehende Befestigungsvorrichtung (2) liegt; die Kniertikulation (4) besteht aus zwei zusammenwirkenden Teilen (6–7); die Zusammenwirkung wird durch Teile erreicht, die um waagerechte, zueinander parallele und voneinander entfernte Achsen (8–9) rotieren; die Fussartikulation (5) besteht aus zwei aufeinanderliegenden durch ein waagerechtes Scharnier (16) verbundenen Gliedern (3–15) und aus zwei zur Pfostenachse (1) waagerechten Druckfedern (20–21), die aber in Bezugnahme auf das Scharnier (16) entgegenwirkende Spannungen ausüben; die Knie- und Fussartikulationen (4–5) sind dabei durch Vorrichtungen miteinander verbunden, die bestehen aus: einem Pfosten (1), einem in dem Pfosten (1) liegenden Rohr, einer an einem Träger (10) befestigten Übertragungsstange (65), einer in dem röhrenförmigen Pfosten (1) eingesetzten Zugstange (26) und einem mit der Zugstange verbundenen und in dem Rohr (28) gleitenden Kolben (27); solche Prothese ist wie folgt gekennzeichnet:

– die Knieartikulationsteile (4–6–7) sind als aufeinanderliegende Hebel gestaltet; solche Hebel sind um die Achsen (8–9) des Trägers (10) hinter der Protheseachse YY artikuliert; der Träger (10) ist röhrenförmig und den zwei Gliedern gemeinsam; dabei sind die Hebelarme mit dem obersten Pfostenende beziehungsweise der Basis der Schenkelsbefestigungsvorrichtung (2) verbunden; die Artikulationsachsen umgebenden Hebelenden (8–9) sind dabei mit ineinandergreifenden Verzahnungen (11–12) versehen;

– die zwei Fussartikulationsdruckfedern (5–10–21) befinden sich die erstere (21) vor und die letztere (20) hinter dem Scharnier (16) als die vordere Druckfeder (21) und sie befindet sich zwischen den beiden Gliedern (3–15), so dass sie sie voneinander entfernt;

– der Kolben (27) der Verbindungsvorrichtungen der Knie- und Fussartikulationen (4–5) ist durch einen Ansatz (30) gekennzeichnet, der nach der Pfostenvorderseite (21) vorspringt; mit dem Ansatz (30) ist ein zur Zugstange (26) waagerechter Schaft (32) verbunden; dabei trägt der Schaft die sich vor dem Fussartikulationsscharnier (5–16) befindende Druckfeder (21), die sich in einer das oberste Artikulationsglied (5–15) durchquerenden Höhlung (23) befindet und dessen Basis sich auf dem untersten Artikulationsglied (3) stützt;

– das oberste Zugstangenende (26) ist mit dem röhrenförmigen Träger (10) verbunden;

– dabei treibt eine in dem Rohr (26) liegende Druckfeder (63) den Kolben (27) gegen die Fussartikulation (5) an.

2. Prothese nach Patentanspruch 1, wie folgt gekennzeichnet: die genannte Übertragungsstange (65) ist so ellengebogen, dass ihr oberstes Ende gegen die Beinhinterseite gewandt ist; dabei geht die Übertragungsstange durch eine Öffnung (6b) des unteren Knieartikulationsglieds (6); und dabei wird der Kopf der Übertragungsstange (65) an dem Träger (10) um eine zur Achse (8) des unteren Hebels (6) waagerechte Achse (62) artikuliert; dabei befindet sich der Kopf der Übertragungsstange auf derselben Ebene wie die Beinlängsachse YY und der Fuss der Übertragungsstange liegt in dem Innenpfosten (1), so dass die Übertragungsstange (65) sich mit der Zugstange (26) ausrichtet.

3. Prothese nach Patentanspruch 1, dadurch gekennzeichnet, dass das oberste Zugstangenende an der Übertragungsstange um eine zur Achse (8) waagerechte Achse (66) artikuliert ist, und dass dessen unteres Ende an dem oberen Ende eines Rohres (68) zusammengeschraubt ist, dessen Basis an dem Kolben (27) befestigt ist.

4. Prothese nach Patentanspruch 1, dadurch gekennzeichnet, dass der genannte Schaft (32) in den Ansatz (30) eingeschraubt ist.

5. Prothese nach Patentanspruch 1, dadurch gekennzeichnet, dass die genannte Druckfeder (21) unten an einem frei in einer Höhlung (23′) des Fussartikulationsglieds (3) liegenden Kopf (34) befestigt ist.

## Claims

1. Articulated prothesis for the lower extremity equipped with articulations (4–5) at the knee and foot level, respectively located at the upper and the lower ends of a tubular vertical jamb (1), on top of which a device (2) for the fastening at the thigh is located, prothesis whose knee articulation (4) consists of two elements (6–7) cooperating with oneanother by means of parts respectively revolving around horizontal axes (8–9) which are parallel to and spaced out from oneanother and whose foot articulation (5) consists of two elements (3–15) placed over oneanother linked by means of a horizontal hinge (16) and of two compression springs (20–21) parallel to the axis of jamb (1) but exerting antagonistic efforts in relation to hinge (16), the knee and foot articulations (4–5) being assembled with oneanother by means of devices consisting of jamb (1), of a tube located inside jamb (1), of a little connecting rod (65) linked to a bearing (10), of a draught (26) inserted into the tubular jamb (1) and of a piston (27) linked to draught (26) sliding inside tube (28) and caracterized as follows:

– the elements (6–7) of the knee articulation (4) have the shape of levers placed over oneanother, articulated around the axes (8–9) behind the axis (YY) of the prothesis on the bearing (10) which is tubular and common to the two elements, the arms of the mentionned levers being linked respectively to the top of jamb (1) and to the bottom of device (2) for the fastening at the thigh and the ends of the mentionned levers, surrounding the articulation axes (8–9), having toothings (11–12) serrating to oneanother;

– the two compression springs (20–21) of foot articulation (5) are placed, the former (21) in front of and the latter (20) behind hinge (16), the spring (20) located behind being weaker than the one in front (21) and being located between the two elements (3–15) in order to keep them apart;

– the piston (27) of the assembling devices between the knee and foot articulations (4–5) requires an appendix (30) leaning out towards the front of jamb (1) to which a stem (32) is linked which is parallel to the draught (26) and carries the spring (21) which is located in front of hinge (16) of the foot articulation (5), which is placed inside a hollow (23) located across the upper element (15) of the foot articulation (5) and whose bottom rests on its lower element (3);

– the upper end of the draught (26) is linked to the tubular bearing (10) by means of a little connecting rod (65), and

– a compression spring (63) placed inside the tube (28) drives the piston (27) towards the foot articulation (5).

2. Prothesis according to claim 1, caracterized by the fact that the little connecting rod (65) mentionned above is elbowed with its upper end aiming at the backside of the leg and that it goes through an opening (6b) of the lower element (6) of the knee articulation and that the head of the little connecting rod (65) is articulated on the bearing (10) around an axis (62) parallel to the axis (8) of the lower lever (6) and which is located on the same plane as the longitudinal axis (YY) of the leg and that the foot of the little connecting rod is located inside the jamb (1) so that the little connecting rod itself (65) is aligned with the draught (26).

3. Prothesis according to claim 1, caracterized by the fact that the upper end of draught (26) is articulated to the little connecting rod (65) around an axis (66) parallel to axis (8) and by the fact that its lower end is screwed into the upper part of a tube (68) whose bottom is fastened to the piston (27).

4. Prothesis according to claim 1, caracterized by the fact that the stem (32) mentionned above is screwed into appendix (30).

5. Prothesis according to claim 1, caracterized by the fact that compression spring (21) mentionned above is fastened at its lower part to a head (34) located free inside o'notch (23') in the element (3) of the foot articulation.